# EUROPÄISCHE PATENTSCHRIFT

(11) **EP 1 140 254 B1**
(45) Veröffentlichungstag und Bekanntmachung des Hinweises auf die Patenterteilung: **04.09.2002**
(21) Anmeldenummer: 00900503.4
(22) Anmeldetag: 05.01.2000
(51) Int. Cl.: A61M 5/00

(54) **SELBSTZERSTÖRENDE EINMALSPRITZE**
SELF-DESTRUCTING ONE-USE SYRINGE
SERINGUE AUTODESTRUCTRICE A USAGE UNIQUE

(30) Priorität: 07.01.1999 DK 1099
(43) Veröffentlichungstag der Anmeldung: 10.10.2001
(73) Patentinhaber: Oeresundshoej Medico Aps., 2920 Charlottenlund (DK)
(72) Erfinder: HETTING, Mikael, DK-2920 Charlottenlund (DK)
(74) Vertreter: Fleck, Thomas, Dr. Dipl.-Chem.
(86) Internationale Anmeldenummer: EP0000028
(87) Internationale Veröffentlichungsnummer: WO00040280

(56) Entgegenhaltungen:
- EP-A- 0 692 272
- DE-A- 19 518 807
- US-A- 5 084 017
- US-A- 5 624 408

## Beschreibung

Die vorliegende Injektionsspritze ist eine Injektionsspritze zum einmaligen Gebrauch, die aus einem Zylinder, einer Kolbenstange und einem Kolben besteht.

Der Zylinder hat am Boden einen Auslauf zur Kanüle und besitzt am oberen offenen Ende einen Kragen, der bei der Bedienung als Fingergriff dient, ausserdem hat der Zylinder auf der Innenseite zwei entgegengesetzte Wülste.

Die Kolbenstange hat eine beilförmige Spitze, die in Zusammenarbeit mit dem, aus Gummimaterial bestehenden Kolben, zur Füllung und Leerung der Spritze dient und zwar so, dass sich die Spritze nur einmal füllen und leeren lässt.

Dies geschieht durch das mechanische Zusammenkoppeln der beilförmigen Kolbenstange und des oberen inneren Vorsprung des Kolbens während der Füllung. Beim Leeren zwingt die beilförmige Spitze der Kolbenstange, den Kolben um 90°C herum und somit weg von der äussersten Spitze der Kolbenstange, die mechanische Zusammenkopplung zwischen Kolben und Kolbenstange wird somit beendet.

Es ist bekannt, dass die effektivste Methode zur Verhinderung von Ansteckung die ist, die Spritzen nur zu einer einzigen Injektion zu verwenden. Es gibt leider mehrere Situationen in denen die Verbraucher hinsichtlich einer Wiederverwendung motiviert sind, dazu gehören unter anderem die Kosten beim Kauf der Spritzen, die in Verbindung mit z.B. Vaccinationskampagnen von grossen Bevölkerungen in armen Gebieten dazu führen kann, dass man das Risiko einer Anstekkung in Kauf nimmt.

Die in der Einleitung beschriebene Injektionsspritze kann nach einer Leerung nicht wieder angewendet werden, da der Kolben sich beim Leeren, durch Druck der Kolbenstange auf den Zylinderboden, 90°C dreht und somit nicht mehr in der Position steht, um mit der Kolbenstange mechanisch zusammengekoppelt zu sein. Somit konfiguriert, während der Leerung der Kolben und die Kolbenstange zu einer neuen Position, ohne mechanische Zusammenkopplung. Die Kolbenstange ist durch die zwei entgegengesetzten Wülste des Zylinders fixiert, im Gegensatz zum horizontal beweglichen Kolben, der sich frei im Zylinder drehen kann, wenn dies vertikal durch Druck der beilförmigen Kolbenstange beeinflusst wird.

Die bekannten Spritzen dieser Art haben jedoch den generellen Nachteil, dass sie wesentlich komplizierter und demnach auch teurer in der Herstellung sind, als konventionelle Spritzen. Ausserdem sind bekannte Spritzen dieser Art im Stande, in verschiedenen Durchgängen gefüllt und geleert zu werden, bis das gesamte Volumen aufgebraucht ist, was ein wesentlicher Nachteil ist. Die oben beschriebene Injektionsspritze kann, laut Erfinder, nur einmal gefüllt und geleert werden, indem die Wirkung der Kolbenstange in Richtung Zylinderboden die Spritze, nach Leerung unanwendbar macht.

Ein Beispiel der bekannten Spritzen ist in US-A-5,624,408 beschrieben. Der Oberbegriff des Anspruches 1 entspricht der Veröffentlichung dieses Dokumentes.

Der Zweck der Spritze der vorliegenden Erfindung ist, eine zum einmaligen Gebrauch gesicherte Injektionsspritze herzustellen die sich, im Verhältnis zu einer konventionellen Spritze, mit minimalen Mehrkosten herstellen lässt. Diese Erfindung hat eine konventionelle Insulinspritze mit integrierter Kanüle als Ausgangspunkt. Durch kleine Änderungen des Kolbens und der Kolbenstange, so wie in Anspruch 1 beschrieben ist, ist es gelungen, mit geringem Aufwand, eine konventionelle Einmalspritze in eine gesicherte Injektionsspritze zu ändern.

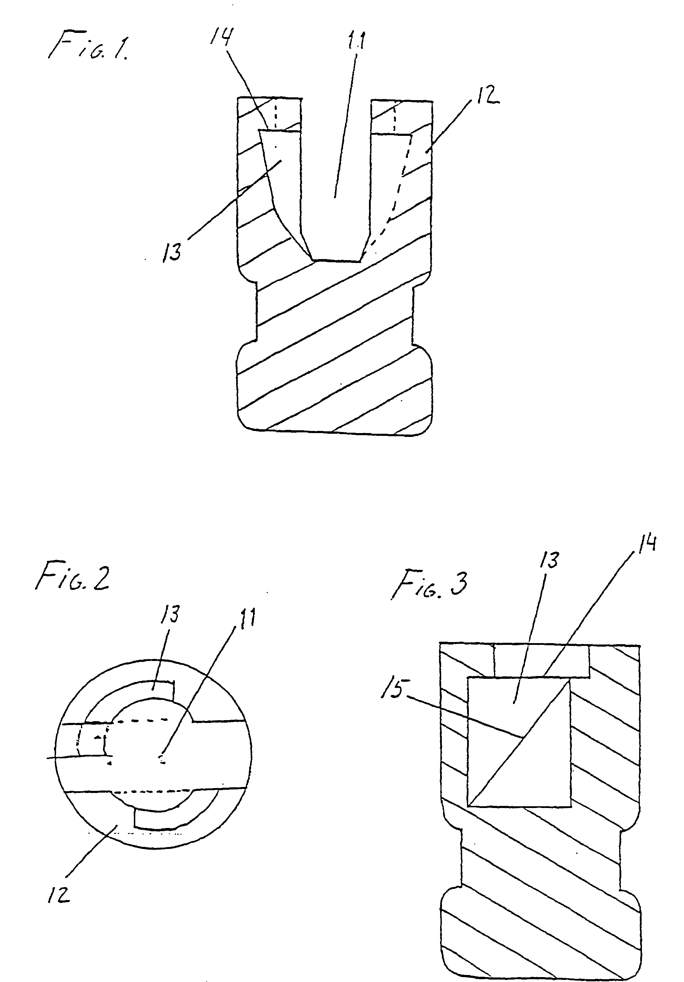

Erfindungsgemäß ist der Kolben nach Fig. 1 durch einen offenen Schlitz 11 gekennzeichnet. Dieser Schlitz wird durch zwei gegeneinander gerichtete Kolbenseiten 12 hervorgerufen, wobei das Innere des Kolbens mit einem speziell geformten Hohlraum 13 versehen ist, der dazu führt, dass sich ein innerer Vorsprung 14 bildet. Dieser Vorsprung wirkt zusammen mit der äusseren Spitze 19 der Kolbenstange, beim Füllen der Spritze.

Der Hohlraum 13 beider Kolbenseiten ist mit schrägen Seitenwänden 15 versehen, die sich nach links neigen und zur Schlitzöffnung 11 drehen. Die Position der Kolbenseiten 12 und somit die Schrägen Seitenwände 15, ergeben zusammen eine Rotation des Kolbens, da bei der Leerung die äusserste Spitze 19 der Kolbenstange auf die schrägen Wände 15 trifft und dem zu Folge den Kolben zur Rotation zwingt.

Dies lässt sich nur dadurch erreichen, dass die Kolbenstange sich nicht rotieren lässt, da diese keine horizontale Drehung machen kann, auf Grund der zwei entgegengesetzten Wülste 20 des Zylinders. Nach beendigter 90°C Rotation sind der Kolben und die Kolbenstange mechanisch permanent von einander abgekoppelt.

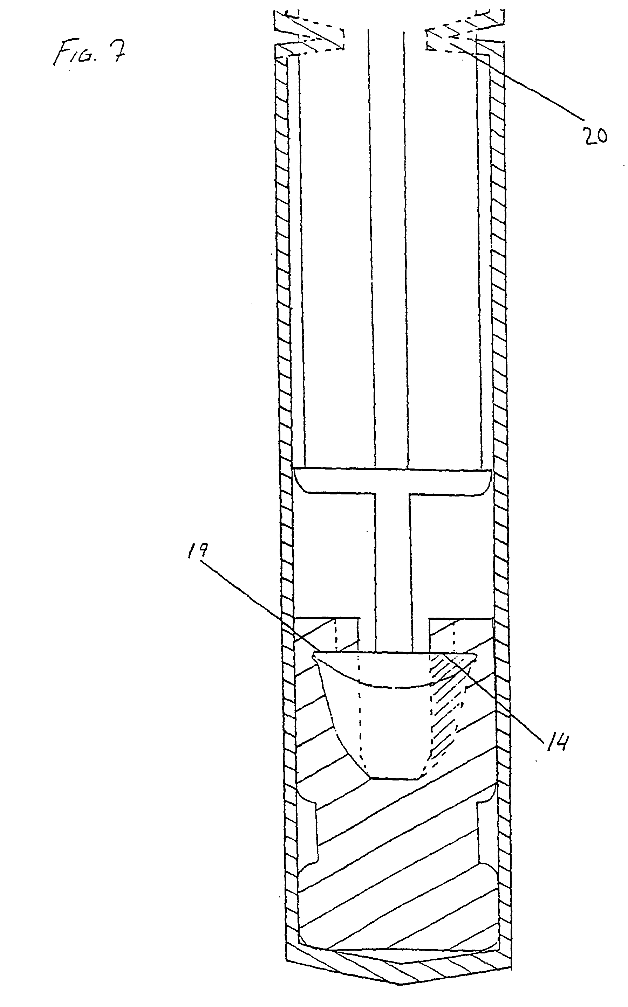

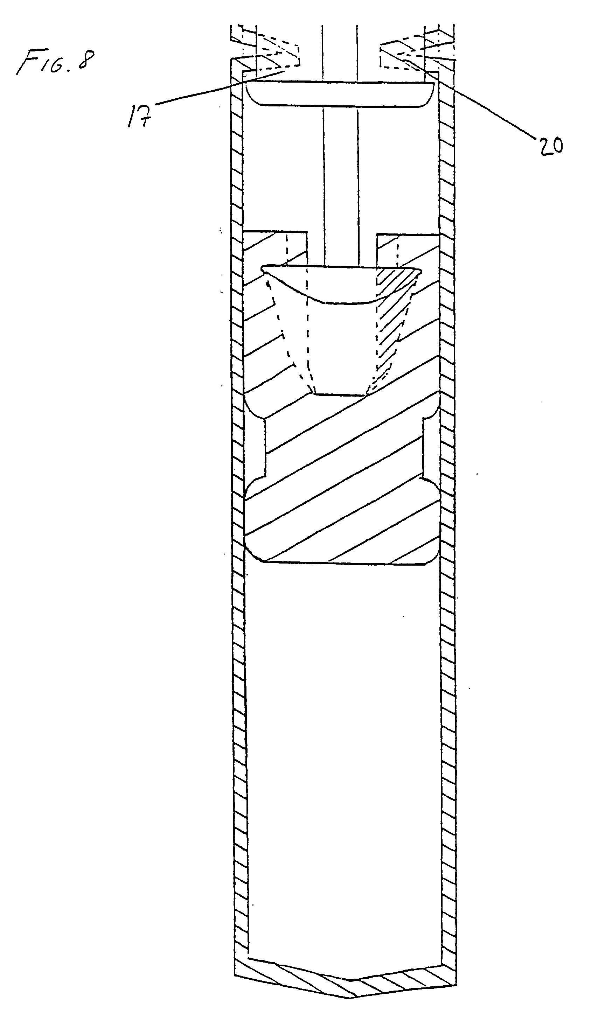

Laut Erfinder ist das Kennzeichen der Kolbenstange Fig. 4 bei dieser Injektionsspritze, dass diese in Plastik gegossen ist und eine glatte Trennfläche hat. Die Kolbenstange ist an den wesentlichen Stellen mit vier Aussparungen 17, sowie einer beilförmigen Spitze 18, deren äusserste Spitze 19 bei der Füllung mit dem Kolben 14 zusammengekoppelt ist, versehen. Die abgerundete Form der Kolbenstange 18 macht die Montage des Kolbens an der Kolbenstange möglich, nachdem der Kolben Fig. 1 am Boden des Zylinders montiert ist. Wenn man die Kolbenstange 18 von oben betrachtet Fig. 6a erscheint diese in rechtwinkliger Form, von unten betrachtet Fig. 6b abgerundet, um die Rotation bei Leerung der Spritze zu erleichtern. Zwei der vier Aussparungen 17 dienen zur mechanischen Zusammenkopplung zusammen mit den zwei gegensätzlichen Wülsten Fig. 7,20 des Zylinders, um die horizontale Drehung der Kolbenstange auszuschliessen.

Laut Erfinder ist der Zylinder dieser Injektionsspritze konventionell, abgesehen von den genannten Wülsten, Fig. 7,20. Nach der mechanischen Montierung des Kolbens und der Kolbenstange werden diese Wülste, durch Erwärmung und Prägung, am Zylinder positioniert.

Eigenheiten in der Ausführung der Spritze, laut Erfinder:
- der Zylinder ist im wesentlichen ungeändert
- der Kolben ist imstande sich während des Leerungsprozesses zu drehen, auf Grund der Wirkung der Kolbenstange
- im Gegensatz zu anderen gesicherten Spritzen, hat diese die gleiche Anzahl Teile wie eine konventionelle, nicht gesicherte Spritze.

Die Erfindung wird nachfolgend näher beschrieben, unter Hinweis auf die entsprechenden Zeichnungen:
- Fig. 1: zeigt Querschnitt des Kolbens
- Fig. 2: zeigt Querschnitt des Kolbens von oben
- Fig. 3: zeigt Innenseite des Kolbens
- Fig. 4: zeigt Kolbenstange mit beilförmiger Spitze

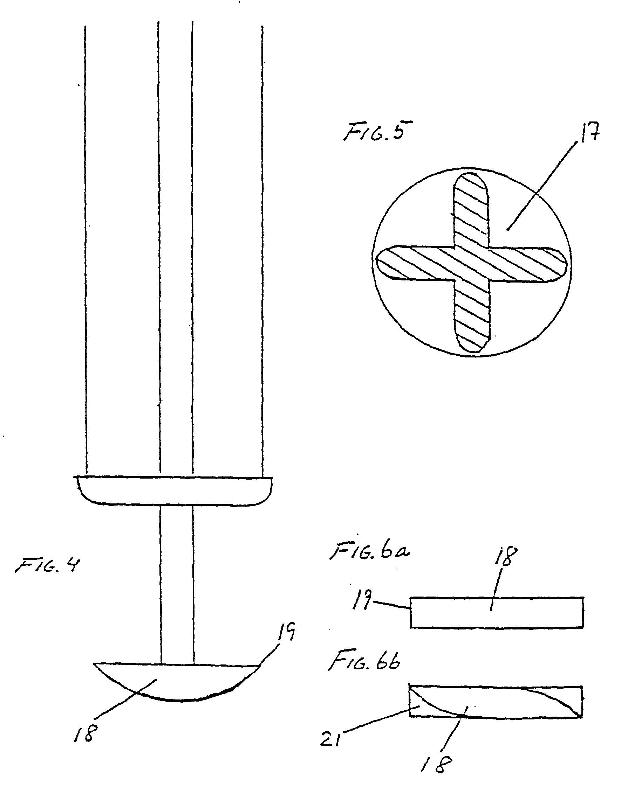

- Fig. 5: zeigt Querschnitt der Kolbenstange mit Aussparungen von oben
- Fig. 6a: zeigt Kolbenstange mit beilförmiger Spitze von oben
- Fig. 6b: zeigt Kolbenstange mit beilförmiger Spitze von unten
- Fig. 7 bis 10: zeigt Füllungs- und Leerungsvorgang

Fig. 7 laut Erfinder, eine fertig montierte Spritze. Die Spritze ist maschinell gefertigt und bei dem gleichen Prozess werden die gegeneinander gerichteten Wülste etabliert, die zur Ausschaltung von horizontalen Bewegungen der Kolbenstange führen. Die Spritze ist klar zur Füllung.

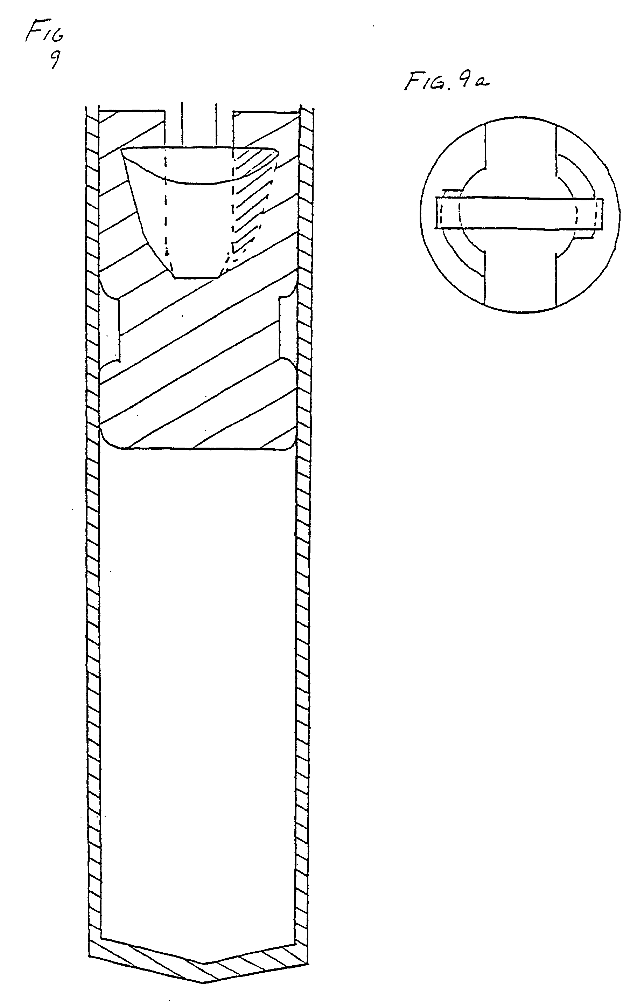

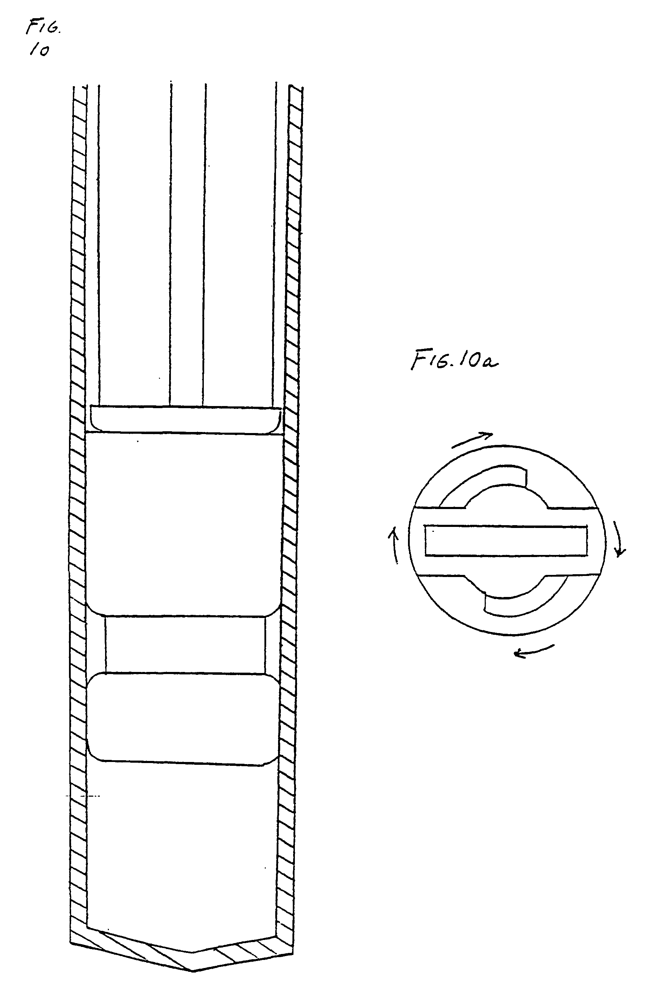

Fig. 8 und Fig. 9 zeigen die Füllung der Spritze. Fig. 9a zeigt wie der Kolben und die Kolbenstange bei der Füllung konfiguriert werden. Fig. 10 zeigt eine Leerungssekvenz, wobei der Kolben von der beilförmigen Spitze der Kolbenstange in die Rotation gezwungen wird und sich 90°C nach rechts dreht. Fig. 10 a zeigt das gleiche nur horizontal.

## Patentansprüche

1. Injektionsspritze zum einmaligen Gebrauch, umfassend:
einen Zylinder, der am Boden einen Auslauf zur Kanüle und am oberen Ende einen Kragen besitzt, der bei Gebrauch der Spritze als Fingergriff dient, einen Kolben, eine Kolbenstange, die beim Füllen der Spritze durch Kupplungsmittel mechanisch mit dem Kolben verbunden wird, wobei eine äußere Spitze der Kolbenstange beim Leeren der Spritze den Kolben zur Rotation zwingt, indem die Spitze (19) durch Druck auf schräge Flächen (15) des oberen Teils des Kolbens den Kolben zur Rotation zwingt, wobei der Zylinder zwei oder mehrere gegeneinander versetzteinnere Vorsprünge oder Wülste (20) besitzt, die mit zwei oder mehreren gegeneinander versetzten Aussparungen (17) der Kolbenstange zusammenwirken,um eine horizontale Drehung der Kolbenstange auszuschließen und ein Herausziehen derselben aus dem Zylinder auszuschließen, und wobei die kolbenstange permanent spätestens bei der Beendigung der Leerung vom Kolben getrennt wird,
**dadurch gekennzeichnet, daß** der Kolben mit einem Hohlraum (13) in einem oberen Innenteil versehen ist, der durch einen Schlitz (11) in mindestens zwei Kolbenseiten (12) unterbrochen ist, wobei die schrägen Flächen (15) in diesem Hohlraum (13) vorgesehen sind und die zwei oder mehreren gegeneinander versetzten inneren Vorsprünge oder Wülste (20) des Zylinders nach der mechanischen Montage von Kolben und Kolbenstange durch Erwärmung und/oder Prägung am Zylinder positioniert werden.

2. Injektionsspritze nach Anspruch 1, **dadurch gekennzeichnet, daß** das obere Innenteil kreiszylindrisch oder nicht-kreiszylindrisch ist.

3. Injektionsspritze nach Anspruch 2, **dadurch gekennzeichnet, daß** das nicht-kreiszylindrische obere Innenteil einen durchgehenden Schlitz (11) aufweist, durch den die Kolbenseiten (12) geteilt werden.

4. Injektionsspritze nach Anspruch 1 bis 3, **dadurch gekennzeichnet, daß** das zylindrische Areal des Kolbens in Zusammenarbeit mit dem Inneren des Zylinders einen Reibungskoeffizienten aufweist, der mehr als 300 kpa Druck auf der Kanülenseite benötigt, um den Kolben auf die Zylinderöffnung zu bewegen.

5. Injektionsspritze nach Anspruch 1 bis 4, **dadurch gekennzeichnet, daß** die Kolbenstange (Fig.4) mit einer axtförmigen Spitze (18) versehen ist, deren äußerste Spitze (19) bei Füllung der Spritze mechanisch mit dem Kolben (14) zusammengekoppelt wird.

6. Injektionsspritze nach Anspruch 5, **dadurch gekennzeichnet, daß** die axtförmige Spitze (18) (von oben gesehen), rechtwinklig oder fast rechtwinklig (Fig.6a) ist und deren Durchmesser von Spitze zu Spitze gleich ist mit dem größten inneren Durchmesser des Kolbens, von unten (Fig.6b) gesehen, wobei die axtförmige Spitze mit abgerundeten Ecken (21) versehen ist, um die Rotation des Kolbens zu erleichtern.

## Claims

1. Injection syringe for one-time use, comprising a cylinder which has on the bottom thereof an outflow to the cannula and has, on the top and thereof a collar serving as a finger grip when using the syringe, the piston shaft and the piston are mechanically during filling connected by coupling means, and whereby an extreme tip of the piston shaft when emptying the syringe forces the piston to rotate, when the tip (19) forces the piston by pressure onto slanted services (15) of the upper part of the piston to rotate the piston whereby the cylinder comprises two or more offset inner projections or bulges (20) which interact with two or more offset recesses (17) of the piston shaft **characterized in that** the piston is provided with a cavity (13) in an upper inner part which is separated by a slit (11) in at least two piston sides (12) whereby the slanted surfaces (15) are provided in said cavity (13) and the two or more offset projections or bulges (20) of the cylinder are positioned after assembly of piston and piston shaft by heating and/or embossing at the cylinder in order to prevent a horizontal rotation of the piston shaft and a pullout of the cylinder thereof and which is permanently separated from the piston at the latest when emptying is ceased.

2. **Syringe according to claim 1,**
**characterized in that** the upper inner part is circular cylindrical or non-circular cylindrical.

3. **Syringe according to claim 2,**
**characterized in that** the non-circular cylindrical upper inner part comprises a slit (11) passing through by which the piston sides (12) are separated.

4. **Syringe according to claim 1 to 3,**
**characterized in that** the piston's cylindrical area in coaction with the internals of the cylinder gives a friction coefficient, which requires more than 300 kpa pressure on the needleside to move the piston towards the opening of the cylinder.

5. **Syringe according to claim 1 to 4,**
**characterized in that** the piston rod (fig. 4) is provided with an axe shaped tip (18) who's outer tips (19) are mechanically coupled with the piston (14) during filling of the syringe.

6. **Syringe according to claim 1 to 5,**
**characterized in that** the axe shaped tip (18) is rectangular or roughly rectangular seen from above fig. 6a, and who's diameter from tip to tip is equal to the largest inner diameter of the piston, and seen from below (fig. 6b) the axe shaped tip is provided with rounded corners (21) to ease the rotation of the piston.

## Revendications

1. Seringue d'injection à usage unique comprenant : un cylindre qui comporte au fond une sortie vers la canule et, à l'extrémité supérieure, une collerette qui sert de prise de doigt pour l'utilisation de la seringue, un piston, une tige de piston qui, pendant le remplissage de la seringue, est reliée mécaniquement au piston par un moyen d'accouplement, où une pointe extérieure de la tige de piston force le piston à effectuer une rotation pendant que la seringue se vide, la pointe (19) forçant le piston à effectuer une rotation par la pression sur des surfaces inclinées (15) de la partie supérieure du piston, où le cylindre est pourvu de deux ou plusieurs épaulements ou renflements (20) intérieurs décalés les uns par rapport aux autres qui agissent en coopération avec deux ou plusieurs encoches (17) de la tige de piston décalées les unes par rapport aux autres pour exclure une rotation horizontale de la tige de piston et pour exclure une extraction de ladite tige hors du cylindre, et où la tige de piston est séparée du piston de façon permanente au plus tard lorsque le vidage se termine, **caractérisée en ce que** le piston est pourvu d'une cavité (13) dans une partie intérieure supérieure qui est interrompue par une fente (11) sur au moins deux côtés de piston (12), où les surfaces inclinées (15) sont prévues dans cette cavité (13) et les deux ou plusieurs épaulements ou renflements (20) intérieurs décalés les uns par rapport aux autres du cylindre sont positionnés, après le montage mécanique du piston et de la tige de piston, par chauffage et/ou estampage sur le cylindre.

2. Seringue d'injection selon la revendication 1, **caractérisée en ce que** la partie intérieure supérieure est cylindrique circulaire ou non cylindrique circulaire.

3. Seringue d'injection selon la revendication 2, **caractérisée en ce que** la partie intérieure supérieure non cylindrique circulaire comporte une fente (11) traversante à travers laquelle les côtés du piston (12) sont séparés.

4. Seringue d'injection selon les revendications 1 à 3, **caractérisée en ce que** l'aire cylindrique du piston, en coopération avec l'intérieur du cylindre, présente un coefficient de friction qui nécessite une pression supérieure à 300 kpa du côté de la canule pour déplacer le piston dans l'ouverture du cylindre.

5. Seringue d'injection selon les revendications 1 à 4, **caractérisée en ce que** la tige de piston (figure 4) est munie d'une pointe en forme de hache (18) dont l'extrême pointe (19) est couplée mécaniquement avec le piston (14) lors du remplissage de la seringue.

6. Seringue d'injection selon la revendication 5, **caractérisée en ce que** la pointe en forme de hache (18) (vue de dessus) est rectangulaire ou presque rectangulaire (figure 6a) et son le diamètre de pointe à pointe est égal au diamètre intérieur le plus grand du piston, vu de dessous (figure 6b), et la pointe en forme de hache est pourvue d'angles arrondis (21) pour faciliter la rotation du piston.
